# EUROPEAN PATENT SPECIFICATION

(11) **EP 1 948 611 B1**
(45) Date of publication and mention of the grant of the patent: **23.05.2012**
(21) Application number: 06819233.5
(22) Date of filing: 03.11.2006
(51) Int. Cl.: C07D 215/18

(54) **Process for the preparation of a leukotriene antagonist and intermediates thereof**
Verfahren zur Herstellung eines Leukotrienantagonisten und seiner Zwischenprodukte
Procédé de préparation d'un antagoniste de leukotriène et ses composés intermédiaires

(30) Priority: 04.11.2005 EP 05110348; 04.11.2005 US 733553 P
(43) Date of publication of application: 30.07.2008
(73) Proprietor: ESTEVE QUIMICA, S.A., 08024 Barcelona (ES)
(72) Inventor: COPPI, Laura, I-20091 Bresso (IT); BARTRA SANMARTÍ, Martí, E-08024 Barcelona (ES); GASANZ GUILLÉN, Yolanda, E-08024 Barcelona (ES); MONSALVATJE LLAGOSTERA, Montserrat, E-08024 Barcelona (ES); TALAVERA ESCASANY, Pedro, E-08024 Barcelona (ES)
(74) Representative: ZBM Patents
(86) International application number: PCT/EP2006/068052
(87) International publication number: WO 2007/051828

(56) References cited:
- US-A1- 2005 234 241

## Description

The present invention relates to a process for the preparation of Montelukast, as well as to some new intermediates useful in such preparation process.

### BACKGROUND ART

Montelukast, is the International Non-proprietary Name (INN) of 1-[[[(1R)-1-[3-[(1E)-2-(7-chloro-2-quinolinyl)ethenyl]phenyl]-3-[2-(1-hydroxy-1-methylethyl)phenyl]propyl]sulfanyl]methyl]cyclopropaneacetic acid, and CAS No. 158966-92-8. Montelukast monosodium salt (CAS No 151767-02-1) is currently used in treatment of asthma, inflammation, angina, cerebral spasm, glomerular nephritis, hepatitis, endotoxemia, uveitis and allograft rejection.

The structure of Montelukast monosodium salt corresponds to formula (I):

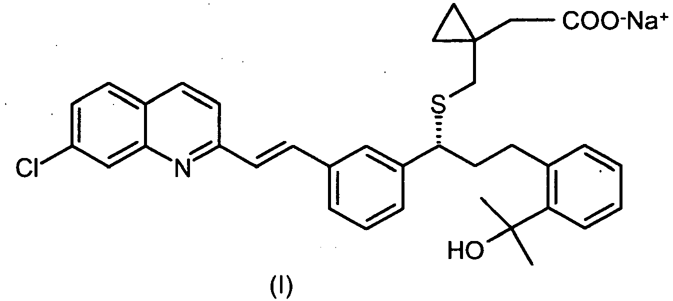

Different synthetic strategies for the preparation of Montelukast and its salts are known. EP 480.717 discloses certain substituted quinolone compounds including Montelukast sodium salt, methods for their preparation, and pharmaceutical compositions using these compounds. Several preparation processes of Montelukast sodium are reported in this document. Example 161 relates to the preparation of Montelukast sodium salt. According to this Example, preparation of Montelukast sodium salt proceeds through its corresponding methyl ester, whose preparation comprises sodium hydride or cesium carbonate assisted coupling of methyl-1-(mercaptomethyl)-cyclopropaneacetate with the protected mesylate (2-(2-(2-(3(S)-(3-(2-(7-chloro-2-quinolinyl)-ethenyl)phenyl)-3-(methanesulfonyloxy)propyl)phenyl)-2-propoxy)tetrahydropyran, generated *in situ*. The methyl ester thus obtained is hydrolyzed to the Montelukast free acid which is then converted directly to the sodium salt. This process is not particularly suitable for large scale production because it requires tedious chromatographic purification of the methyl ester intermediate and/or the final product, and yields of intermediates and final product are low. Another process for the preparation of Montelukast sodium salt comprises the reaction of the thioacetate with the following formula: 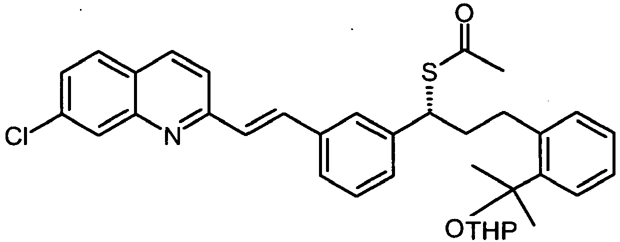 with hydrazine or an alkoxide, followed by reaction with an appropriate 1-substituted cyclopropyl acetate, followed by deprotection of the tertiary alcohol to afford Montelukast.

EP 737.186 relates to a process for the preparation of Montelukast or its salts thereof, which comprises reacting the dilithium dianion of 1-(mercaptomethyl)cyclopropaneacetic acid with the corresponding mesylate alcohol ((2-(2-(2-(3(S)-(3-(2-(7-chloro-2-quinolinyl)-ethenyl)phenyl)-3-(methanesulfonyloxy)propyl)phenyl)-2-propanol), to obtain Montelukast, which is further converted to the corresponding sodium salt via dicyclohexyl amine salt.

CN 1.420.113 A relates to a process for the preparation of Montelukast or its sodium salt thereof, which comprises the reaction of the thioacetic acid ester with the following formula: 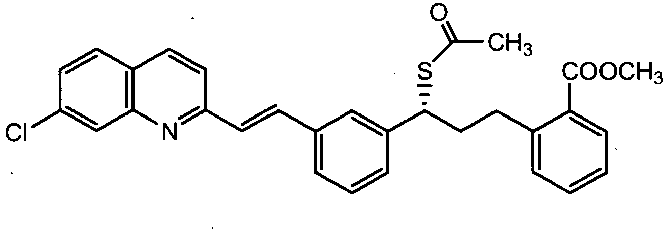 and methyl magnesium iodide to afford the thiol alcohol with the following formula:

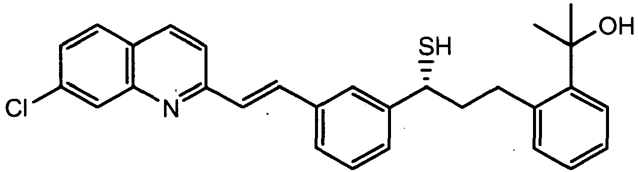

Montelukast is synthesized by reaction between the mentioned above thiol alcohol intermediate and appropriate 1-halo substituted cyclopropyl acetate, followed by hydrolysis of the ester group.

US 2005107612 describes a process for the preparation of Montelukast or a salt thereof, which comprises reacting a late intermediate compound which is 2-[1-[1-R-3-[2-(7 chloro quinolin-2-yl) vinyl [phenyl]-3-[2-methoxy carbonyl phenyl] propyl sulfonyl methyl] cyclo propyl] acetic acid or a salt thereof with methyl magnesium chloride or methyl magnesium bromide.

Finally, US 2005/0234241 describes a process for the preparation of Montelukast based on the reaction of 2-(2-(3(S)-3-(2-(7-chloro-2-quinolinyl)ethenyl)phenyl)-3-sulfonyl oxy propyl)phenyl)-2-propanol with a salt of 1-(mercaptomethyl)cyclopropane acetonitrile or of 1-(mercaptomethyl)cyclopropane acetamide, followed by an hydrolysis reaction with a base.

Although certain processes for the preparation of Montelukast are known, there continue being a need for new processes for the preparation of Montelukast and its salts.

### SUMMARY OF THE INVENTION

Inventors have found a new efficient preparation process of Montelukast from new intermediate compounds, which proceeds with high yields and without the need of chromatographic purifications.

Thus, according to one aspect of the present invention, it is provided a preparation process of Montelukast (I), or a pharmaceutically acceptable salt, or a solvate thereof, including a hydrate, 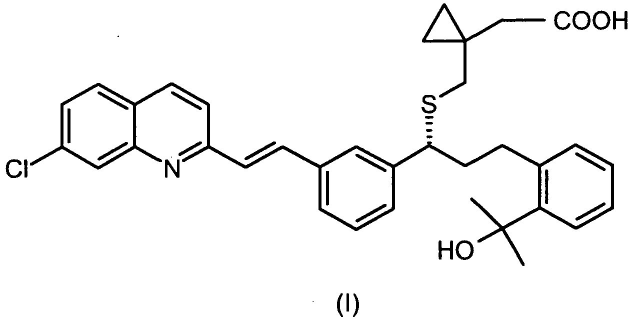 which comprises the steps of: (a) reacting a compound of formula (IV) wherein R₁ is a radical selected from (C₁-C₄)-alkyl, phenyl, and phenyl mono- or disubstituted by a (C₁-C₄)-alkyl radical, with an alkaline metal cyanide, to give a compound of formula (III) and isolating said compound (III) as free base or as a salt thereof; . 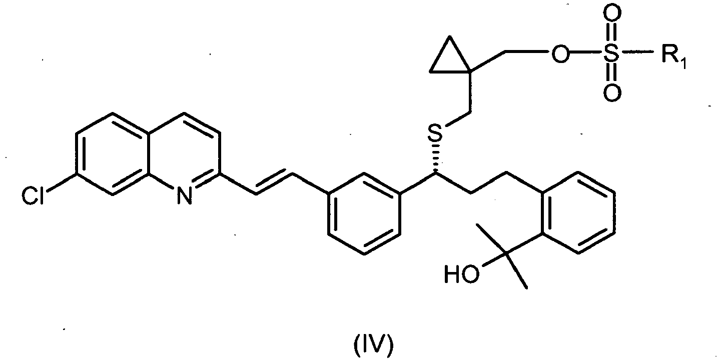 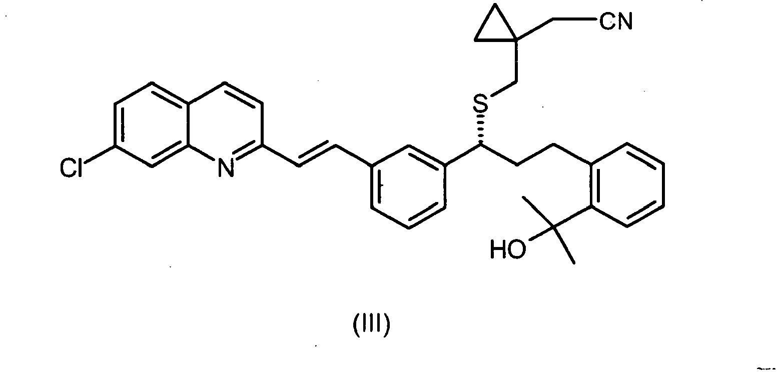
(b) Optionally, hydrolysing the compound of formula (III) obtained in step (a) in reaction conditons that lead to a compound of formula (II) and isolating said compound (II) from the reaction medium; 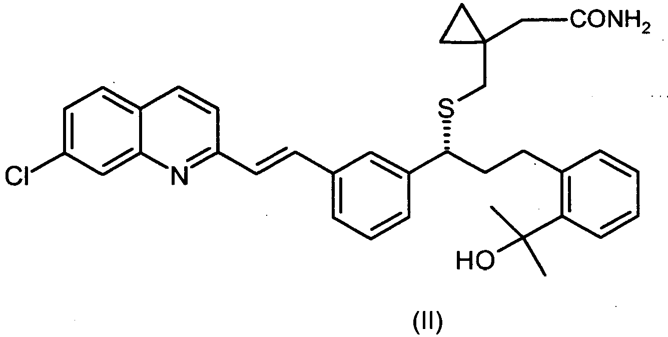
(c) submiting the compound obtained in step (a) or in step (b) to an hydrolysis reaction in reaction conditons that lead to Montelukast (I); and (d) optionally treating Montelukast (I) with a pharmaceutically acceptable base to form the corresponding salt.

Previously, in a further step of the preparation process, the alcohol of formula (V) is reacted with a sulphonyl chloride of formula Cl-SO₂-R₁, wherein R₁ has the same meaning mentioned above, to give the compound of formula (IV).

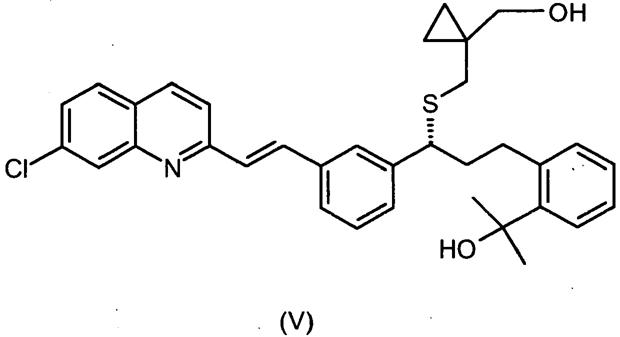

In another further step, the compound of formula (VI) is reacted with a Grignard reagent selected from methyl lithium and methyl magnesium halide, optionally in the presence of cerium chloride, to give the compound of formula (V) that can be isolated as free base or as a salt thereof.

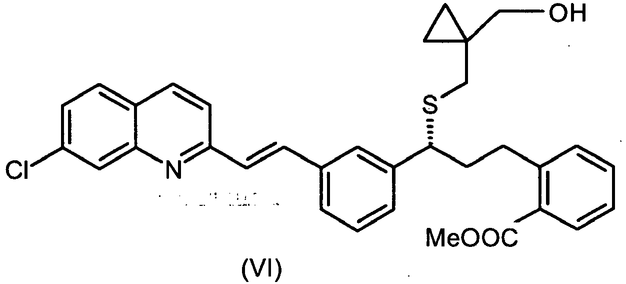

In another further step, the compound of formula (VII) where R₂ is a radical selected from (C₁-C₄)-alkyl, phenyl, and phenyl mono- or disubstituted by (C₁- . C₄)-alkyl radicals, is reacted with a compound of formula (VIII), in the presence of a base selected from cesium carbonate, sodium hydroxide, and lithium bis(trimethylsilyl)amide, to give the compound of formula (VI) that can be isolated as free base or as a salt thereof.

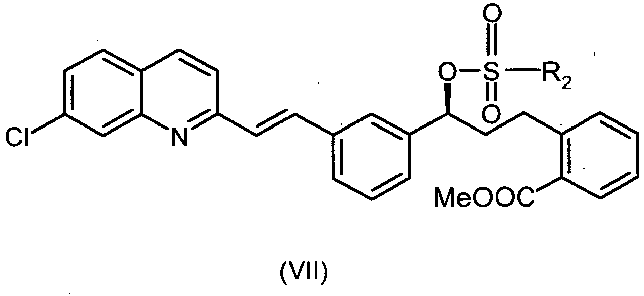

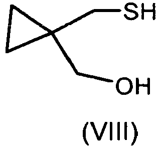

The above defined compounds of formula (IV), (V) or its salts, and (VI) or its salts are new and are also part of the invention. Thus, another aspect of the present invention is to provide new intermediate compounds for the preparation of Montelukast, in particular those of formula (IV), (V) and (VI). It also forms part of the invention the salts of compound (V), in particular the citrate salt, the salts of compound (VI), in particular the oxalate salt, and the salts formed by the compound of formula (III) with oxalic acid, L(-)-malic acid, L-(+)-tartaric acid, maleic acid, fumaric acid, succinic acid, benzoic acid, 4-hydroxymandelic acid, citric acid, benzensulfonic acid and mandelic acid.

The process of the present invention is particularly advantageous in its practical industrial realization because is cost effective and suited for scale up. Unlike other known processes for the preparation of Montelukast, this process avoids intramolecular cyclizations of the intermediate compounds. Thus, all intermediates are formed cleanly, and therefore chromatographic separations were not required. Furthermore, the final product is obtained in high chemical and optical purity and with high yields.

### DETAILED DESCRIPTION OF THE INVENTION

As it is described above, the cyano compound of formula (III) as free base or as a salt thereof is obtained from the sulfonate of formula (IV). Preferably, the compound of formula (III) is obtained as free base from the compound of formula (IV). The conversion is carried out by reaction of the compound (IV) with an alkaline metal cyanide such as sodium or potassium cyanide, in an appropriate solvent such as dimethylformamide, dimethylsufoxide, ethyl acetate or acetonitrile, and at a temperature comprised between 0°C and reflux temperature. Preferably, the reaction is carried out at about 60 °C. The reaction can be carried out in the presence of a phase transfer catalyst. Example of suitable phase transfer catalysts are tri-C₈-₁₀-alkylmethylammonium chlorides, tetrabutylammonium bromide, hexadeciltrimethylammonium chloride, methyltrioctilammonium chloride, tetrabutylammonium chloride or tetrabutylammonium hydrogen sulfate. In a preferred embodiment the tetrabutylammonium hydrogen sulfate is used as phase transfer catalyst.

In the present invention, preferred sulfonate compounds of formula (IV) are those where the sulfonate (IV) is a mesylate (R₁= methyl), a besylate (R₁= phenyl) or a tosylate (R₁= 4-methylphenyl). The most preferred sulfonate (IV) is the mesylate.

The compound of formula (III) can be converted into its salts, and its salts can be converted into the free compound by methods known in the art.

The amide compound of formula (II) can be prepared from the cyano compound of formula (III) as free base or as a salt by hydrolysis. Preferably, the compound of formula (II) is obtained from the compound of formula (III) as free base. For instance, hydrolysis can be achieved using mild conditions and/or by shortening the reaction time.The conditions to carry out the hydrolysis may be easily determined by the person skilled in the art by routine tests. For example, the hydrolysis can be carried out using the same reacting conditions for carrying out the hydrolysis of the cyano compound to Montelukast but reducting the reaction time, as it illustrated in Example 9, followed by the isolation of the amide compound (II).

The last step of the process is a hydrolysis reaction. The compound of formula (II) can be hydrolyzed to afford Montelukast. The hydrolysis reaction can also be directly performed from the cyano compound (III) as free base or as a salt to afford Montelukast. Preferably, the hydrolysis of the compound of formula (II) or of the compound of formula (III) as free base or as a salt is carried out with a base. Preferably the base is an alkaline metal hydroxide or an alkaline earth metal hydroxide. More preferably, the base is sodium hydroxide, potassium hydroxide or lithium hydroxide. The most preferred one is sodium hydroxide. The reaction can be carried out in different solvent systems. Preferably the solvent system is a solvent mixture comprising C₁-C₄ alcohol and water. Preferably the C₁-C₄ alcohol is ethanol or isopropanol. The most preferred one is ethanol. Typically, the hydrolysis of the cyano compound (III) to montelukast (I) is carried out at reflux temperature and it is essentially complete within 24 hours.

Other suitable solvent system can be a two-phase solvent mixture comprising water and a suitable organic solvent non-miscible in water or sparingly miscible in water, optionally in the presence of a phase transfer catalyst. Preferably the organic solvent is a (C₆-C₈)-aromatic hydrocarbon. The most preferred one is toluene. Suitable phase transfer catalyst include for instance, an ammonium quaternarium salt such as tetrabutylamonium bromide, tri-C₈-C₁₀-alkylmethylammonium chlorides, methyltrioctilammonium chloride or hexadeciltrimethylammonium chloride. The hydrolysis can be carried out at a temperature comprised between room temperature and reflux temperature. Typically, in these reaction conditions the hydrolysis of the cyano compound (III) to montelukast (I) is carried out at 120 °C and it is essentially complete within about seven days.

The isolation of crude Montelukast can be accomplished by diluting the crude with toluene and washing the solution with aqueous acetic acid and susbsequently with water at room temperature, followed by evaporation of the solvent. The obtained Montelukast can be purified by several methods, for instance, by aqueous acid-base extractions or by recrystallization.

Montelukast free acid obtained by the process of the present invention may be converted into pharmaceutically acceptable salts, and salts may be converted into free acid compounds, by known methods described in the art. It is also possible to isolate a salt of Montelukast from the hydrolysis reaction, i.e. without isolating the montelukast free acid.

The sulfonate compounds of formula (IV) are prepared from the alcohol of formula (V) by reaction with the corresponding sulfonyl chloride. This reaction is carried out in an appropriate solvent and in the presence of a tertiary amine, at a temperature comprised between -20 °C and room temperature. Preferably, the reaction is carried out at low temperatures. Common solvents for the reaction include chlorine-containing solvents such as methylene chloride or 1,2-dichloroethylene, aromatic hydrocarbons such as toluene or xylene, and dimethylformamide. Examples of suitable tertiary amines are diisopropylethylamine and triethylamine.

The preparation of the alcohol of formula (V) as free base or as a salt from the compound of formula (VI) is carried out by reaction with a methyl magnesium halide such as methyl magnesium chloride or methyl magnesium bromide, optionally in the presence of cerium chloride, or by reaction with methyl lithium, in the presence of a suitable solvent. Examples of suitable solvents include an ether such as tetrahydrofurane, or an aromatic hydrocarbon such as toluene or xylene, or mixtures of them. Preferably the reaction is carried out at a temperature comprised between -78°C and 20 °C, , more preferably, at -20 °C.

The alcohol of formula (V) can be converted into its salts, and its salts can be converted into the free compound by methods known in the art. For instance, the citrate salt is prepared by reaction of the compound of formula (V) as free base with citric acid in the presence of a suitable solvent.

The compound of formula (VI) as free base or as a salt can be prepared from the known compound of formula (VII), which is reacted with the compound of formula (VIII) in a suitable solvent such as dimethylformamide, dimethylsulfoxide, dichloromethane, toluene or tetrahydrofurane, and in the presence of a base such as cesium carbonate, sodium hydroxide and lithium bis(trimethylsilyl)amide. Preferably, the reaction is carried out at a temperature comprised between -10 °C and 60 °C, more preferably, at a 0-5°C.

The compound of formula (VI) can be converted into its salts, and its salts can be converted into the free compound by methods known in the art. For instance, the oxalate salt is prepared by reaction of the compound of formula (VI) as free base with oxalic acid in the presence of a suitable solvent.

The compound of formula (VII) may be prepared according to a known process described in US 2005107612. The process described in this document comprises the reaction of the methyl-2-((S)-3-(3-((E)-2-(7-chloroquinolin-2-yl)vinyl)phenyl)-3-hydroxypropyl)benzoate of formula (IX) with methane sulfonyl chloride or toluene sulfonyl chloride to form the methyl-2-((S)-3-(3-(E)-(2-(7-chloroquinolin-2-yl)vinyl)phenyl)-3-[(methylsulfonyl)oxy]propyl)benzoate or the corresponding tosylate. Analogously, the besylate of formula (VII) can be obtained from the corresponding benzene sulfonyl chloride.

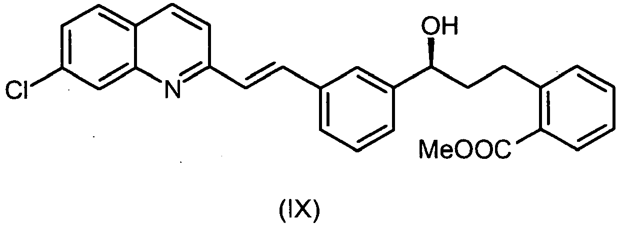

The compound of formula (VIII) can be prepared from 5.7-dioxa-6-thiaspiro[2.5]octane 6-oxide according to the process summarized in Scheme 1.

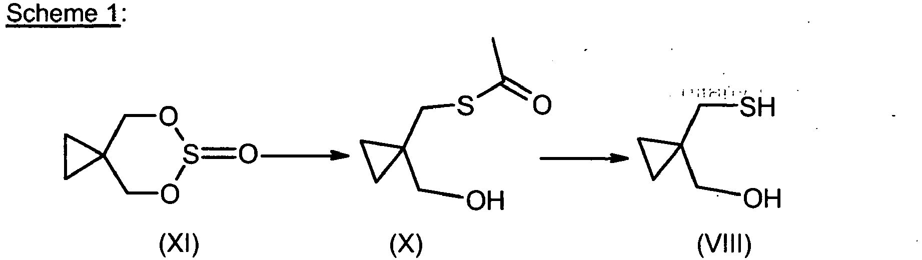

The preparation process of the compound of formula (VIII) involves the reaction of the compound of formula (XI) with potassium ethanethioate to give the compound of formula (X). Typically, such reaction is carried out with an excess of potassium ethanethiolate in an appropriate solvent. Examples of suitable solvents include dimethylsulfoxide, dimethylformamide, ethyl acetate, acetonitrile or mixtures thereof. Preferably the reaction is carried out at a temperature comprised between room temperature and 70°C. After the isolation of the compound of formula (X), it can be hydrolyzed using an acid or a basic catalyst to give the compound of formula (VIII). Examples of suitable acids are hydrochloric acid, sulfuric acid, acetic acid and formic acid. Examples of suitable bases are hydroxides, carbonates and alcoxide of an alkaline or an earth alkaline metal. Example of suitable solvents are (C₁-C₆)-alcohols, (C₆-C₈)-aromatic hydrocarbons, dimethylformamide, dimethylsulfoxide, or mixtures of them.

The best conditions to carry out the process of the present invention vary according to the parameters considered by a person skilled in the art, such as the starting materials, molar ratio, temperature, and similar. Such reaction conditions may be easily determined by a person skilled in the art by routine tests, and with the teaching of the examples included in this document.

Throughout the description and claims the word "comprise" and variations of the word, are not intended to exclude other technical features, additives, components, or steps. The abstract of this application is incorporated herein as reference. Additional objects, advantages and features of the invention will become apparent to those skilled in the art upon examination of the description or may be learned by practice of the invention. The following examples are provided by way of illustration, and they are not intended to be limiting of the present invention.

### EXAMPLES

### Example 1: Preparation of S-(1-(hydroxymethyl)cyclopropyl)methyl ethanethioate (X)

200 g of 5,7-dioxa-6-thiaspiro[2.5]octane 6-oxide were dissolved in 1.2 I of dimethyl sulfoxide and 308 g of potassium ethanethioate were poured to the solution. Then, the suspension was heated at 40°C for 5. Once the reaction was completed, a combination of 3.6 I of ethyl acetate and 3.6 I of water was added. The organic phase was separated, washed with water and concentrated to dryness. 200 g of S-(1-(hydroxymethyl)cyclopropyl)methyl ethanethioate were recovered. Yield: 78% corrected by GC. ¹H NMR (400 MHz, CDCl₃): 3.45 (2H, d, J: 6.4 Hz); 3.01 (2H, s); 2.53 (OH, broad triplet, J: 6.4 Hz); 2.39 (3H, s); 0.54 (4H, m).

### Example 2: Preparation of (1-(mercaptomethyl)cyclopropyl)methanol (VIII)

200 g of S-(1-(hydroxymethyl)cyclopropyl)methyl ethanethioate were dissolved in 2 I of methanol. Then, 111 ml of concentrated HCl were added under a nitrogen blanket and the solution was stirred at room temperature for 10. The solvent was distilled off and the residue was re-dissolved in 1.5 I of dimethylformamide to be used in the preparation of methyl 2-((R)-3-(3-((E)-2-(7-chloroquinolin-2-yl)vinyl)phenyl)-3-(((1-(hydroxymethyl) cyclopropyl)methyl)sulfanyl)propyl)benzoate. Yield: 100%.¹H NMR (400 MHz, CDCl₃): 3.57 (2H, s); 2.63 (2H, d, J: 8.0 Hz); 2.45 (OH, broad signal,); 1.43 (SH, t, J: 8.0 Hz); 0.52 (4H, m).

### Example 3: Preparation of methyl 2-((S)-3-(3-((E)-2-(7-chloroquinolin-2-yl)vinyl)phenyl)-3-[(methylsulfonyl)oxy]propyl)benzoate (VII with R₂: CH₃)

12.1 ml de diisopropylethylamine were poured to a stirred solution of 24.5 g of methyl 2-((S)-3-(3-((E)-2-(7-chloroquinolin-2-yl)vinyl)phenyl)-3-hydroxypropyl)benzoate in 120 ml of dichloromethane. The mixture is cooled to -20 °C and 5 ml of mesyl chloride were added slowly. Once the reaction was completed, the crude solution was successively washed with 120 ml of an aqueous 10% NaHCO₃ solution and 120 ml of water. Finally, the solvent was distilled off to obtain 29 g of the title compound. The product was re-dissolved in 290 ml of dimethylformamide to be used as a solution in the next step. Yield: 100%.

### Example 4: Preparation of methyl 2-((R)-3-(3-((E)-2-(7-chloroquinolin-2-yl)vinyl)phenyl)-3-(((1-(hydroxymethyl)cyclopropyl)methyl)sulfanyl)propyl) benzoate (VI)

A solution of 19 g of (1-(mercaptomethyl)cyclopropyl)methanol in 190 ml of dimethylformamide was cooled to 0 °C. Then, 52 g of Cs₂CO₃ were added in one portion. After 10 min, the outcome solution from the previous example was added and the suspension was maintained at the same temperature for 18 hours. Finally, a mix of 380 ml of ethyl acetate and 380 ml of water was poured to the solution crude. The organic phase was washed with 380 ml of water and concentrated to dryness. 30 g of brown oil were recovered. Yield: 86%, corrected after HPLC analysis. ¹H NMR (400 MHz, DMSO-d₆): 8.38 (1H, d, J: 8.4 Hz); 8.00-7.26 (14H, m); 4.50 (OH, t, J: 5.4 Hz); 3.92 (1H, t, 7.2 Hz); 3.76 (3H, s); 3.33 (1H, dd, J: 11.0 Hz, 5.4 Hz); 3.25 (1H, dd, J: 11.0 Hz, 5.4 , Hz); 2.98-2.88 (1H, m); 2.83-2.74 (1H, m); 2.52 (1H, d, J: 12.7 Hz); 2.39 (1H, d, J: 12.7 Hz); 2.10 (2H, m); 0.46-0.18 (4H, m).

### Example 5: Preparation of 2-(2-((R)-3-(3-((E)-2-(7-chloroquinolin-2-yl)vinyl)phenyl)-3-(((1-(hydroxymethyl)cyclopropyl)methyl)sulfanyl)propyl) phenyl)propan-2-ol (V)

The product obtained in the previous example was dissolved in 300 ml of tetrahydrofuran. The mixture was cooled to 0 °C and 307 ml of 1.4M solution of methylmagnesium bromide were added under a nitrogen blanket. The reaction was maintained at 0 °C for 18 hours. Then, 400 ml of 2M aqueous solution of acetic acid were added slowly, followed of 400 ml of ethyl acetate. The organic phase was successively washed with 400 ml of an aqueous 10% NaHCO₃ solution and 400 ml of water. Finally, the solvent was distilled off to yield 30 g of crude. Yield: 81% corrected after HPLC analysis. ¹H NMR (400 MHz, DMSO-d₆): 8.39 (1H, d, J: 8.6 Hz); 8.00-7.06 (14H, m): 4.91 (OH, s); 4.51 (OH, t, J: 5.5 Hz); 3.99 (1H, t, 7.3 Hz); 3.34 (1H, dd, J: 11.0 Hz, 5.5 Hz); 3.26 (1H, dd, J: 11.0 Hz, 5.5 Hz); 3.05 (1H, m); 2.74 (1H, m); 2.54 (1H, d, J: 12.7 Hz); 2.41 (1H, d, J: 12.7 Hz); 2.15 (2H, m); 1.44 (6H, s); 0.45-0.36 (2H, m); 0.33-0.22 (2H, m).

### Example 6: Preparation of 2-(2-((R)-3-(3-((E)-2-(7-chloroguinolin-2-yl)vinyl)phenyl)-3-(((1-(hydroxymethyl)cyclopropyl)methyl)sulfanyl)propyl) phenyl)propan-2-ol (V)

1.3 g of anhydrous cerium chloride were stirred in 10 ml of dry tetrahydrofuran for 18 hours at room temperature. Then, the suspension was cooled to -20 °C and 1.5 ml of a 3M solution of methylmagnesium chloride were added under a nitrogen blanket. After a period of time, a 2.5 ml tetrahydrofuran solution of 0.5 g of the product obtained in example 4 was added. The mixture was stirred for 18 hours at the same temperature and 10 ml of 2M aqueous solution of acetic acid were added slowly, followed of 10 ml of ethyl acetate. The organic phase was successively washed with 10 ml of an aqueous 10% NaHCO₃ solution and 10 ml of water. Finally, the solvent was distilled off to yield 0.5 g of crude. Yield: 80% corrected after HPLC analysis.

### Example 7: Preparation of (R,E)-(1-((1-(3-(2-(7-chloroquinolin-2-yl)vinyl)phenyl)-3-(2-(2-hydroxypropan-2-yl)phenyl)propyl)sulfanyl)methyl) cyclopropyl)methyl methanesulfonate ((IV) with R₁= CH₃)

6.5 ml de diisopropylethylamine were poured to a stirred solution of 16 g of 2-(2-((R)-3-(3-((E)-2-(7-chloroquinolin-2-yl)vinyl)phenyl)-3-(((1-(hydroxymethyl)cyclopropyl)methyl)sulfanyl)propyl)phenyl)propan-2-ol in 160 ml of dichloromethane. The mixture was cooled to -20 °C and 2.7 ml of mesyl chloride were added slowly. The mixture was stirred at the same temperature for one hour and then, it was washed twice with 300 ml of water. The solvent was distilled off and 18 g of crude were recovered. Yield: 100%.¹H NMR (400 MHz, DMSO-d6): 8.41 (1H, d, J: 9.6 Hz); 8.02-7.03 (14H, m); 4.91 (OH, s); 4.12 (2 H, m); 4.02 (1H, t, J: 7.1 Hz); 3.12 (3H, s); 3.09-3.02 (1H, m); 2.78-2.71 (1H, m); 2.54 (1H, d, J: 13.2 Hz); 2.46 (1H, d, J: 13.2 Hz); 2.15 (2H, m); 1.43 (3H, s); 1.42 (3H, s); 0.68-0.45 (4H, m).

### Example 8: Preparation of (R,E)-2-(1-((1-(3-(2-(7-chloroquinolin-2-yl)vinyl)phenyl)-3-(2-(2-hydroxypropan-2-yl)phenyl)propyl)sulfanyl)methyl) cyclopropyl)acetonitrile (III)

The crude obtained in the previous example was dissolved in 180 ml of dimethylformamide and 2.1 g of sodium cyanide were added in one portion to the solution. After 18 hours of stirring at 60 °C, 270 ml of ethyl acetate was poured into the mixture previously warmed to room temperature. Then, two washes with 270 ml of water were carried out and the solvent was distilled off to afford 14 g of crude. Yield: 75% corrected after HPLC analysis. ¹H NMR (400 MHz, DMSO-d₆): 8.39 (1H, d, J: 8.6 Hz); 8.00-7.05 (14H, m); 4.90 (OH, s); 4.04 (1H, t, J: 7.2 Hz); 3.06 (1H, m); 2.76 (1H, m); 2.67 (1H, m); 2.33 (1H, m); 2.30 (2H, s); 2.13 (2H, m); 1.43 (6H, s); 0.59-0.43 (4H, m).

### Example 9: Preparation of (R,E)-2-(1-((1-(3-(2-(7-chloroquinolin-2-yl)vinyl)phenyl)-3-(2-(2-hydroxypropan-2-yl)phenyl)propyl) sulfanyl)methyl)cyclopropyl)acetamide (II)

1.8 g of crude-obtained in example 8 were dissolved in 3.6 ml of toluene. Then, 3.6 ml of water was added followed of 14.7 g of sodium hydroxide and 0.44 g of tetrabutylammonium bromide. The mixture was stirred at 120 °C for 30 hours. After this period of time, 10 ml of toluene and 10 ml of water were added to the reaction solution previously cooled to room temperature. The organic phase was separated and washed with 10 ml of water. Finally, the solvent was distilled off and the crude was purified by standard methods in order to recover 77 mg of the title compound. Yield: 6%.¹H NMR (400 MHz, DMSO-d₆): 8.41 (1H, d, J: 8.6 Hz); 8.03-7.07 (15H, m); 6.74 (1H, s); 4.91 (OH, s); 4.02 (1H, t, J: 7.3 Hz); 3.04 (1H, m); 2.75 (1H, m); 2.57 (1H, d, J: 13.3 Hz); 2.45 (1H, d, J: 13.3 Hz); 2.16 (4H, m); 1.43 (6H, s); 0.50-0.27 (4H, m).

### Example 10: Preparation of 2-(1-((((R)-1-(3-((E)-2-(7-chloroquinolin-2-yl)vinyl)phenyl)-3-(2-(2-hydroxypropan-2-yl)phenyl)propyl)sulfanyl)methyl) cyclopropyl)acetic acid (Montelukast free acid)

5.2 g of crude obtained in example 8 were dissolved in 10 ml of toluene. Then, 10 ml of water was added followed of 14.7 g of sodium hydroxide and 0.44 g of tetrabutylammonium bromide. The mixture was stirred at 120 °C for 7 days. After this period of time, 10 ml of toluene and 10 ml of water were added to the reaction solution previously cooled to room temperature. The organic phase was separated and successively washed with 10 ml of acetic acid and 10 ml of water. Finally, the solvent was distilled off to recover 4.6 g of product, that can be purified by standard methods if it is necessary. Yield: 83%.¹H NMR (400 MHz, DMSO-d₆): 12.02 (1H, s); 8.39 (1H, d, J: 8.6 Hz); 8.01-7.04 (14H, m); 4.90 (OH, s); 4.00 (1H, t, J: 7.3 Hz); 3.05 (1H, m); 2.76 (1H, m); 2.56 (1H, d, J: 12.9 Hz); 2.47 (1H, d, J: 12.9 Hz); 2.32 (2H, s); 2.16 (2H, m); 1.44 (6H, s); 0.52-0.33 (4H, m). IR (KBr) = 3573.1, 3436.9, 2919.2, 1716.9, 1608.9, 1499.9, 1408.8, 1315.7, 1223.9, 1201.6, 1173.0, 1148.0, 1134.9, 1074.8, 965.9, 950.8, 933.2, 863.6, 842.7, 766.0 cm⁻¹.

### Example 11: Preparation of 2-(1-((((R)-1-(3-((E)-2-(7-chloroquinolin-2-yl)vinyl)phenyl)-3-(2-(2-hydroxypropan-2-yl)phenyl)propyl)sulfanyl)methyl) cyclopropyl)acetic acid (Montelukast free acid)

1.05 g of sodium hydroxide were poured to a solution of 1 g of (R,E)-2-(1-((1-(3-(2-(7-chloroquinolin-2-yl)vinyl)phenyl)-3-(2-(2-hydroxypropan-2-yl)phenyl)propyl)sulfanyl)methyl)cyclopropyl)acetonitrile in 5 ml of a mixture of ethanol:water (96:4, v/v). The outcome suspension was stirred at reflux temperature for 15 hours. After this period of time, the reaction mixture was cooled down to room temperature and diluted with 10 ml of toluene. Then, 20 ml of a 2M aqueous solution of acetic acid were added slowly and the organic layer was separated and washed twice with 10 ml of water. Finally, the solvent was distilled off to recover 0.9 g of product that can be purified by standard methods if it is necessary. Yield: 91%.

### Example 12: Preparation of sodium 2-(1-((((R)-1-(3-((E)-2-(7-chloroquinolin-2 yl)vinyl)phenyl)-3-(2-(2-hydroxypropan-2-yl)phenyl)propyl)sulfanyl)methyl) cyclopropyl) acetate (Montelukast sodium)

2.6 g of Montelukast free acid were dissolved in 26 ml of toluene and 8.9 ml of 0.5M NaOH solution in methanol were added slowly at room temperature. The mixture was stirred for 1 hour and the solvent was removed under vacuum to obtain a residue. Then, heptane (24 ml) was added over 30 min to a well stirred solution of the residue in 4 ml of ethyl acetate at room temperature. Two hours after the addition, an off white solid was filtered off under a nitrogen atmosphere and washed with 5 ml of heptane. The wet product was dried under vacuum at 70-80 °C for 2 days to yield 2.7 g of amorphous solid form of Montelukast sodium. Yield: 100%. ¹H NMR (400 MHz, DMSO-d₆): 8.38 (1H, d, J: 8.6 Hz); 8.02-7.04 (14H, m); 5.19 (OH, s); 4.01 (1H, t, J: 7.2 Hz); 3.08 (1H, m); 2.72 (1H, m); 2.69 (1H, d, J: 12.4 Hz); 2.54 (1H, d, J: 12.4 Hz); 2.22 (1H, m); 2.10 (1H, d, J: 14.2 Hz); 2.10 (1H, m); 1.96 (1H, d, J: 14.2 Hz); 1.45 (3H, s); 1.44 (3H, s); 0.46-0.32 (2H, m); 0.28-0.15 (2H, m).

### Example 13: Preparation of oxalate salt of methyl 2-((R)-3-(3-((E)-2-(7-chloroquinolin-2-yl)vinyl)phenyl)-3-(((1-(hydroxymethyl)cyclopropyl)methyl)-sulfanyl)propyl)benzoate (oxalate salt of compound (VI))

To a solution of 338 g (0.61 mol) of methyl 2-((R)-3-(3-((E)-2-(7-chloroquinolin-2-yl)vinyl)phenyl)-3-(((1-(hydroxymethyl)cyclopropyl)methyl) sulfanyl)propyl)benzoate in 2.0 I of toluene a solution of 75 g (0.60 mol) of oxalic acid dihydrate in 1.0 I of acetonitrile was slowly added at room temperature. The outcome suspension was stirred at room temperature during ten hours. The solid was filtered off and washed several times with toluene/acetonitrile 2/1 (v/v) mixture. The wet solid was dried under vacuum at 25°C. 304 g of a yellow solid were obtained. Yield 78%. DSC: 121.6°C (peak). ¹H NMR (400 MHz, DMSO-d6): 8.42 (1H, d, J: 8.6 Hz); 8.00-7.28 (14H, m); 3.92 (1H, t, 7.3 Hz); 3.76 (3H, s); 3.31 (1H, d, J: 11.0 Hz); 3.25 (1H, d, J: 11.0 Hz); 2.98-2.90 (1H, m); 2.82-2.75 (1H, m); 2.52 (1H, d, J: 12.7 Hz); 2.39 (1H, d, J: 12.7 Hz); 2.14-2.08 (2H, m); 0.43-0.19 (4H, m). DSC measurement was carried out in a perforated pan at a scan rate of 5°C/minute from 25.0°C to 150.0°C with a DSC Mettler Toledo DSC822.

### Example 14: Preparation of methyl 2-((R)-3-(3-((E)-2-(7-chloroquinolin-2-yl)vinyl)phenyl)-3-(((1-(hydroxymethyl)cyclopropyl)methyl)sulfanyl)propyl) benzoate (VI)

283 g of the oxalate salt prepared in Example 13 were suspended in 1.7 I of toluene and 1.7 I of water. 35% of aqueous hydrochloric acid solution was slowly added to the previous mixture until pH 6.5 was obtained. The organic phase was separated and washed with water. Finally the solvent was distilled off under vacuum. 243 g of an oil was obtained. Yield 100%.

### Example 15: Preparation of 2-(2-((R)-3-(3-((E)-2-(7-chloroquinolin-2-yl)vinyl)phenyl)-3-(((1-(hydroxymethyl)cyclopropyl)methyl)sulfanyl)propyl) phenyl)propan-2-ol citrate (citrate salt of compound (V))

A solution of 92.5 g of 2-(2-((R)-3-(3-((E)-2-(7-chloroquinolin-2-yl)vinyl)phenyl)-3-(((1-(hydroxymethyl)cyclopropyl)methyl)sulfanyl)propyl) phenyl)propan-2-ol (HPLC: 78 area %) in 910 ml of toluene was heated to 55 °C. Then, 34.8 g of citric acid monohydrate were added in one portion. After 1 hour of stirring at the same temperature, the mixture was gradually cooled to 20 °C in 2 hours and stirred at this temperature for 2 hours more. Finally, the suspension was filtered under vacuum and the cake was washed twice with 100 ml of toluene. The product was used as a wet solid in the next step. (HPLC: 97 area %). ¹H NMR (400 MHz, DMSO-d6): 12.38 (2H, s); 8.39 (¹H, d, J: 8.6 Hz); 8.02-7.11 (14H, m); 4.90 (OH, s); 4.51 (OH, s); 3.99 (1H, t, J: 7.3 Hz); 3.34 (1H, d, J: 11.0 Hz); 3.27 (1H, d, J: 11.0 Hz); 3.06 (1H, td, J: 12.3 Hz; J: 4.9 Hz); 2.77 (2H, d, J: 15.4 Hz); 2.73 (1H, m); 2.66 (2H, d, J: 15.4 Hz); 2.54 (1H, d, J: 12.7 Hz); 2.41 (1H, d, J: 12.7 Hz); 2.17 (2H, m); 1.44 (6H, s); 0.44-0.36 (2H, m); 0.32-0.22 (2H, m). IR (KBr) = 3411, 3052, 2970, 2922, 2593, 1727, 1632, 1599, 1488, 1437, 1409, 1376, 1318, 1223, 1208, 1153, 1080, 1026, 961, 927, 861, 840, 760, 729, 694, 598, 471 cm⁻¹.

### Example 16: Preparation of 2-(2-((R)-3-(3-((E)-2-(7-chloroquinolin-2-yl)vinyl)phenyl)-3-(((1-(hydroxymethyl)cyclopropyl)methyl)sulfanyl)propyl) phenyl)propan-2-ol (V)

The wet solid obtained in Example 15 was partitioned with 800 ml of toluene and 800 ml of an aqueous solution of NaHCO₃ 5%. The organic layer was separated, washed with 800 ml of water and concentrated to dryness by rotary evaporation under vacuum at 30 °C. 29.7 g of a pale yellow solid were recovered. Overall yield for the 2 steps: 39% (HPLC: 96 area %).

### Example 17: Preparation of (R,E)-2-(1-((1-(3-(2-(7-chloroquinolin-2-yl)vinyl) phenyl)-3-(2-(2-hydroxypropan-2-yl)phenyl)propyl)sulfanyl)methyl)cyclopropyl)acetonitrile (III)

5 ml of water were added to a 96 g of a toluene solution containing 10 g of (R,E)-(1-((1-(3-(2-(7-chloroquinolin-2-yl)vinyl)phenyl)-3-(2-(2-hydroxypropan-2-yl)phenyl)propylthio)methyl)cyclopropyl)methyl methanesulfonate; followed of 0.5 g of tetrabutylammonium hydrogen sulfate and 0.85 g of sodium cyanide. The mixture was stirred at 60 °C for 20 hours. Then, it was cooled to room temperature and the organic layer was washed three times with 100 ml of water. Finally, the outcome toluene solution was concentrated to dryness by rotary evaporation under vacuum at 30 °C. 9.5 g were obtained.

### General example: Preparation of salts of (R,E)-2-(1-((1-(3-(2-(7-chloroquinolin -2-yl)vinyl)phenyl)-3-(2-(2-hydroxypropan-2-yl)phenyl)propyl)sulfanyl)methyl) cyclopropyl)acetonitrile (salts of compound (III))

To a solution of 0.5 g (8.82 mmol) of (R,E)-2-(1-((1-(3-(2-(7-chloroquinolin-2-yl)vinyl)phenyl)-3-(2-(2-hydroxypropan-2-yl)phenyl)propyl)sulfanyl)methyl) cyclopropyl)acetonitrile in 3.5 ml of toluene a solution of 8.82 mmol of the acid in acetonitrile or tetrahydrofurane was slowly added at room temperature. The solvent was distilled off and the theoretical amount of the salt was obtained as a yellow solid.

The general example has been repeated using the corresponding acids indicated in the following examples:

Example 18: Preparation of oxalate salt of the compound (III) using oxalic acid. Yield: 100%; IR (KBr) (cm⁻¹): 3420, 2970, 2925, 1719, 1626, 1600, 1486, 1408, 1205, 1157, 1.080, 762, 695; ¹H RMN(400 MHz, DMSO-d6): 8.41 (1H, d, J: 8.7 Hz); 8.0-7.1 (14H, m); 4.04 (1H, t, 7.3 Hz); 3.1 (1H, m); 2.8 (1H, m); 2.63 (2H, s); 2.51 (2H, under semideuterated DMSO); 2.2 (2H, m); 1.44 (3H, s); 1.43 (3H, s); 0.60-0.40 (4H, m).

Example 19: Preparation of malate salt of the compound (III) using L-(-)-malic acid; Yield: 100%; IR (KBr) (cm-1): 3436, 2976, 2930, 1721, 1628, 1607, 1498, 1409, 1161, 1078, 761, 696; ¹H RMN(400 MHz, DMSO-d6): 12.41 (1H, broad); 8.40 (1H, d, J 8.69 Hz); 8.0-7.1 (14H, m); 4.91 (1H, s); 4.26 (1H, dd, J 7.83 Hz, J 4.78 Hz); 4.04 (1H, t, 7.35 Hz); 3.11-3.01 (1H, m); 2.80-2.70 (1H, m); 2.61 (1H, dd, J 15.66 Hz, J 4.80 Hz); 2.51 (2H, under semideuterated DMSO), 2.44 (1H, dd, J 15.66Hz, J 7.84Hz); 2.26-2.10 (2H, m); 1.44 (3H, s); 1.43 (3H, s); 0.60-0.40 (4H, m).

Example 20: Preparation of tartrate salt of the compound (III) using L-(+)-Tartaric acid: Yield: 100%; IR (KBr)(cm-1): 3401, 2970, 2927, 1726, 1626, 1605, 1487, 1408, 1205, 1130, 1080, 762, 696; ¹H RMN(400 MHz, DMSO-d6):12.68 (1H, broad); 8.41 (1H, d, J: 8.64 Hz); 8.04-7.04 (14H, m); 4.91 (1H, s); 4.31 (2H, s); 4.04 (1H, t, J 7.34 Hz); 3.12-3.00 (1H, m); 2.81-2.70 (1H, m); 2.64 (2H, s); 2.51 (2H, under semideuterated DMSO); 2.26-2.07 (2H, m);1.44 (3H, s); 1.43 (3H, s); 0.60-0.40 (4H, m).

The following salts of the compound (IIII) were also prepared using the process of the general example from the corresponding acids: maleate, fumarate, succinate, benzoate, 4-hydroxymandelate, citrate and mandelate.

### Example 21: Preparation of oxalate salt of (R,E)-2-(1-((1-(3-(2-(7-chloroquinolin-2-yl)vinvl)phenyl)-3-(2-(2-hydroxvpropan-2-yl)phenyl) propyl)sulfanyl)methyl)cyclopropyl)acetonitrile (oxalate salt of compound (III)) .

To a solution of 3.14 g (5.53 mmol) of (*R,E*)-2-(1-((1-(3-(2-(7-chloroquinolin-2-yl)vinyl)phenyl)-3-(2-(2-hydroxypropan-2-yl)phenyl)propyl)sulfanyl)methyl)-cyclopropyl)acetonitrile in 21.5 ml of toluene a solution of 0.68 g (5.42 mmol) of oxalic acid dihydrate in 10 ml acetonitrile was added at room temperature. The solution was concentrated under vacuum to half the initial volume and stirred at room temperature overnight. The outcome solid was filtered off and dried under vacuum. 2.71 g (71% yield) of a yellow solid were obtained.¹H NMR (400 MHz, DMSO-d6): 8.41 (1H, d, J: 8.7 Hz); 8.0-7.1 (14H, m); 4.04 (1 H, t, 7.3 Hz); 3.1 (1H, m); 2.8 (1H, m); 2.63 (2H, s); 2.51 (2H, under semideuterated DMSO); 2.2 (2H, m); 1.44 (3H, s); 1.43 (3H, s); 0.60-0.40 (4H, m). IR (KBr) = 3420, 2970, 2925, 1719, 1626, 1600, 1486, 1408, 1205, 1157, 1.080, 762, 695 cm⁻¹.

### Example 22: Preparation of benzensulfonate salt of (R,E)-2-(1-((1-(3-(2-(7-chloroquinolin-2-yl)vinyl)phenyl)-3-(2-(2-hydroxypropan-2-yl)phenyl)propyl)sulfanyl)methyl)cyclopropyl)acetonitrile (benzensulfonate salt of compound (III)

To a solution of 1.0 g (1.76 mmol) of (R,E)-2-(1-((1-(3-(2-(7-chloroquinolin-2-yl)vinyl)phenyl)-3-(2-(2-hydroxypropan-2-yl)phenyl)propy)sulfanyl)methyl)-cyclopropyl)acetonitrile in 6 ml of toluene a solution of 0.303 g (1.73 mmol) of benzensulfonic acid 90% in 0.6 ml acetonitrile was added at room temperature. The solution was stirred at room temperature overnight. The outcome solid was filtered off and dried under vacuum. 0.96 g (75% yield) of a yellow solid were obtained. ¹H NMR (400 MHz, DMSO-d6): 8.74 (1H, d, J: 8.7 Hz); 8.27-7.04 (19H, m); 4.07 (1H, t, 7.3 Hz); 3.1 (1H, m); 2.8 (1H, m); 2.63 (2H, s); 2.51 (2H, under semideuterated DMSO); 2.2 (2H, m); 1.44 (3H, s); 1.43 (3H, s); 0.61-0.41 (4H, m). IR (KBr) = 3430, 3050, 2910, 1625, 1600, 1480, 1230, 1150, 1125, 1020, 990, 690, 610 cm-1

## Claims

1. A preparation process of Montelukast (I), or a pharmaceutically acceptable salt, or a solvate thereof, including a hydrate, 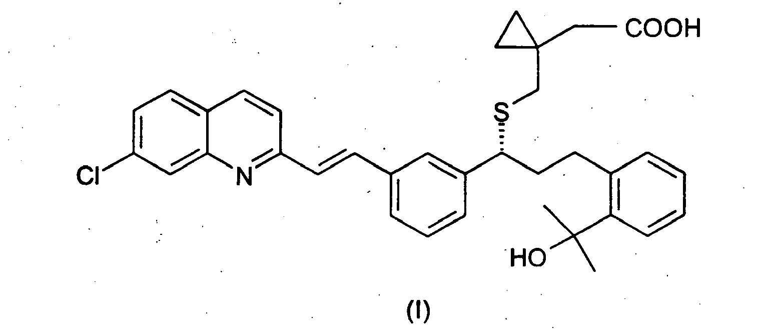 which comprises the steps of:
(a) reacting a compound of formula (IV) wherein R₁ is a radical selected from the group consisting of (C₁-C₄)-alkyl, phenyl, and phenyl mono- or disubstituted by a (C₁-C₄)-alkyl radical, with an alkaline metal cyanide, to give a compound of formula (III) and isolating said compound (III) as free base or as a salt thereof; 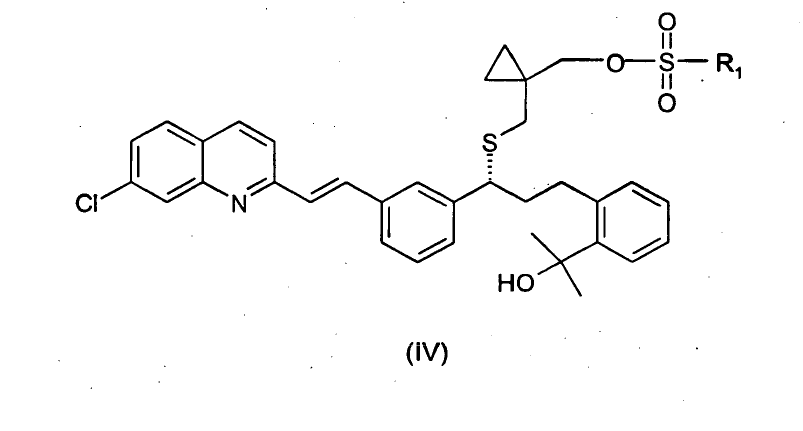 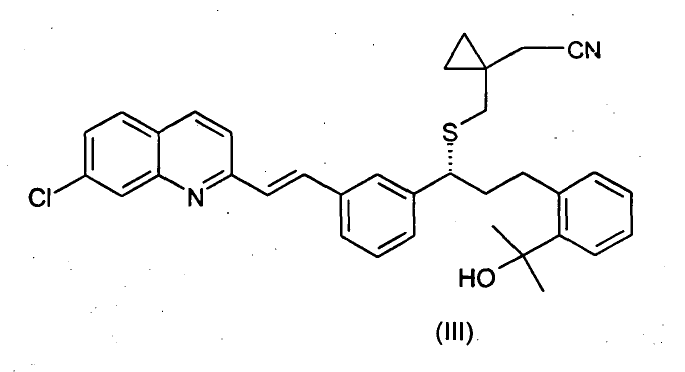
(b) Optionally, hydrolysing the compound of formula (III) obtained in step (a) in reaction conditions that lead to a compound of formula (II) and isolating said compound (II) from the reaction medium; 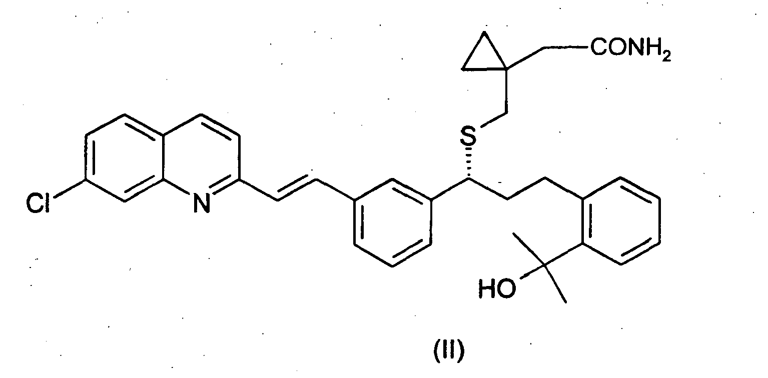
(c) submiting the compound obtained in step (a) or in step (b) to a hydrolysis reaction in reaction conditons that lead to Montelukast (I); and
(d) optionally treating Montelukast (I) with a pharmaceutically acceptable base to form the corresponding salt.

2. The preparation process according to claim 1, wherein the compound of formula (III) is isolated as free base.

3. The preparation process according to any of the claims 1-2, wherein R₁ is selected from the group consisting of methyl, phenyl, and 4-methylphenyl.

4. The preparation process according to any of the claims 1-3, wherein step (a) is carried out in the presence of a phase transfer catalyst.

5. The preparation process according to any of the claims 1-4, wherein the hydrolysis reaction of steps (b) and (c) is carried out with a base.

6. The preparation process according to claim 5, wherein the hydrolysis is carried out into a mixture comprising water and an organic solvent, optionally in the presence of a phase transfer catalyst.

7. The preparation process according to any of the claims 1-6, wherein in a further step a compound of formula (V) is reacted with a sulphonyl chloride of formula Cl-SO₂-R₁, wherein R₁ has the same meaning as in claim 1 to give a compound of formula (IV).

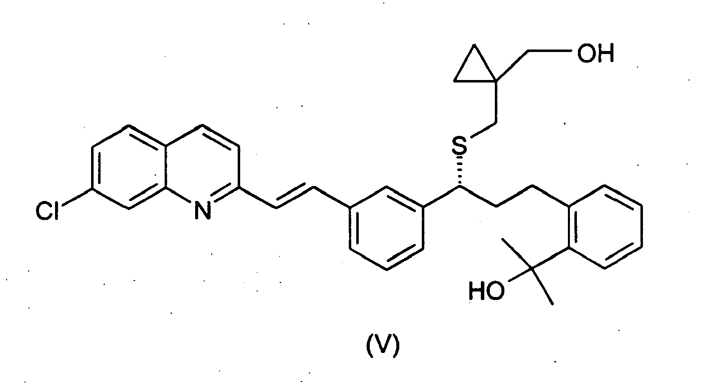

8. The preparation process according to claim 7, wherein in a further step a compound of formula (VI) is reacted with a Grignard reagent selected from methyl lithium and a methyl magnesium halide, optionally in the presence of cerium chloride to give a compound of formula (V) and said compound (V) is isolated as free base or as a salt thereof.

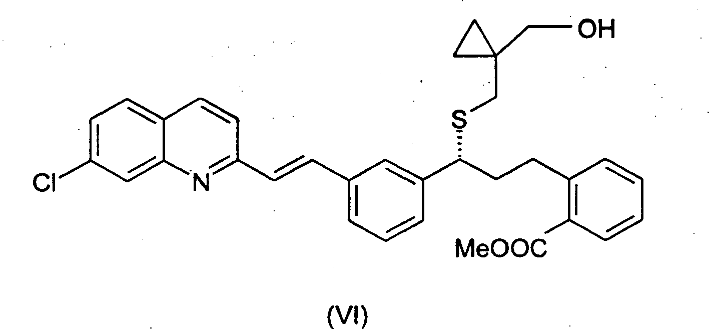

9. The preparation process according to claim 8, wherein the compound of formula (V) is isolated as free base.

10. The preparation process according to any of the claims 8-9, wherein in a further step the compound of formula (VII) wherein R₂ is a radical selected from the group consisting of (C₁-C₄)-alkyl, phenyl, and phenyl mono- or disubstituted by (C₁-C₄)-alkyl radicals, is reacted with a compound of formula (VIII), in the presence of a base to give a compound of formula (VI) and said compound (VI) is isolated as free base or as a salt thereof.

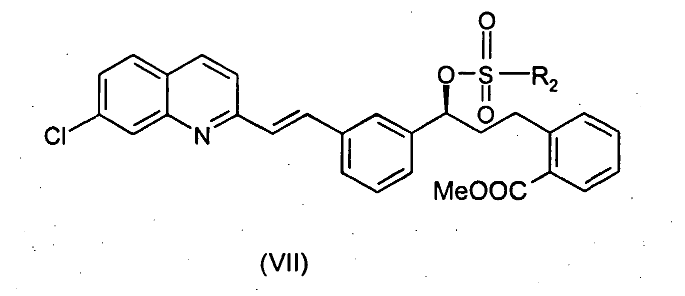

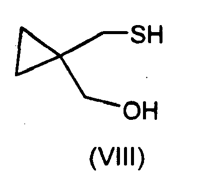

11. The preparation process according to claim 10, wherein the compound of formula (VI) is isolated as free base.

12. The preparation process according to claim 10, wherein the compound (VI) is isolated as the oxalate salt.

13. The preparation process according to any of the claims 10-12, wherein R₂ is selected from the group consisting of methyl, phenyl, and 4-methylphenyl.

14. A compound of formula (IV), wherein R₁ is a radical selected from the group consisting of (C₁-C₄)-alkyl, phenyl, and phenyl mono- or disubstituted by a (C₁-C₄)-alkyl radical.

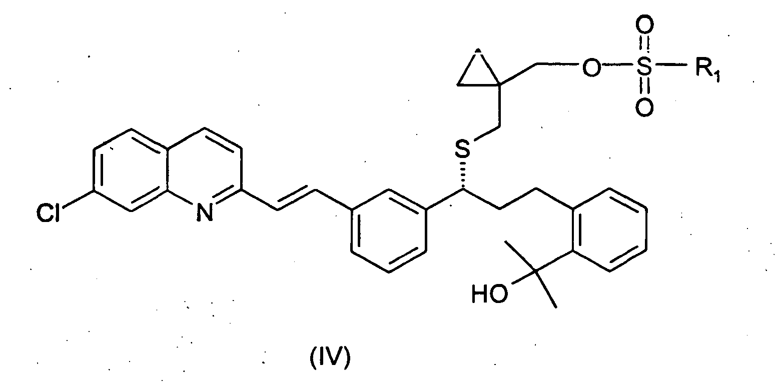

15. The compound according to claim 14, wherein R₁ is selected from methyl, phenyl, and 4-methylphenyl.

16. A compound of formula (V) or a salt thereof.

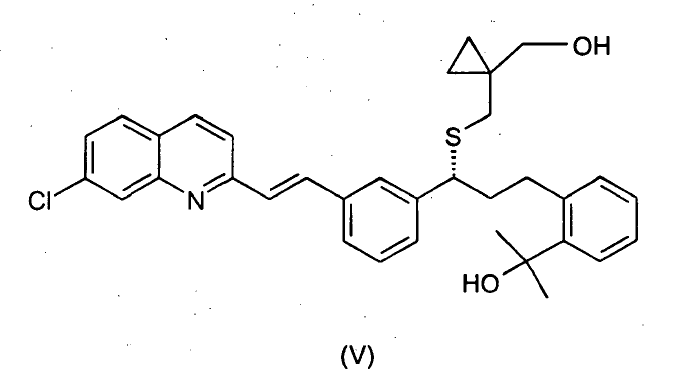

17. The compound according to claim 16, wherein the salt is the citrate.

18. A compound of formula (VI) or a salt thereof.

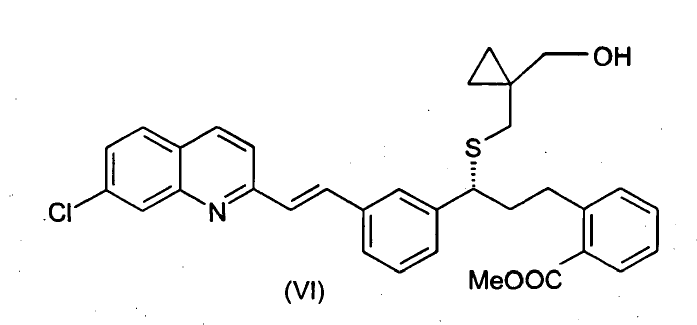

19. The compound according to claim 18, wherein the salt is the oxalate.

20. A salt of the compound of formula (III) which is selected from the group consisting of oxalate, L-(-)-malate, L-(-)-tartrate, maleate, fumarate, succinate, benzoate, 4-hydroxymandelate, citrate, benzenesulfonate, and mandelate.

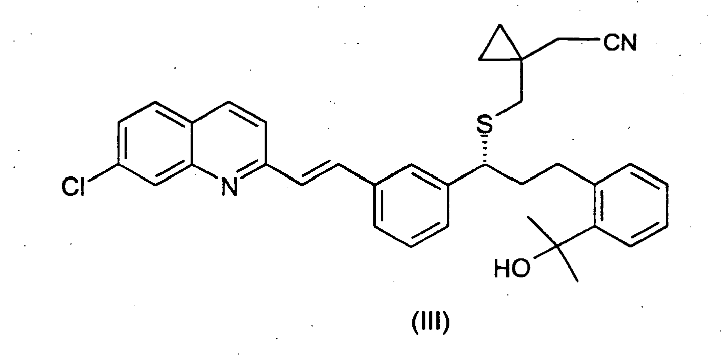

## Patentansprüche

1. Verfahren zur Herstellung von Montelukast (I), oder eines pharmazeutisch verträglichen Salzes, oder eines Solvats davon, einschließlich eines Hydrats, 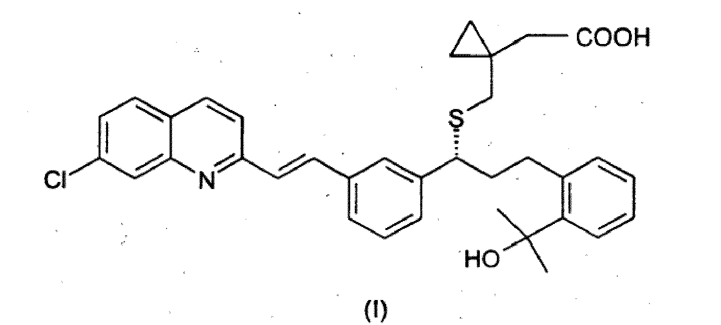 mit folgenden Schritten:
(a) Reaktion einer Verbindung der Formel (IV), worin R₁ ein Radikal ist, das aus der Gruppe, die aus (C₁-C₄)-Alkyl, Phenyl, und ein- oder zweifach mit einem (C₁-C₄)-Alkylradikal substituiertem Phenyl besteht, ausgewählt wird, mit einem Alkalimetallcyanid, um eine Verbindung der Formel (III) zu ergeben, und Isolieren der besagten Verbindung (III) in Form einer freien Base oder eines Salzes davon; 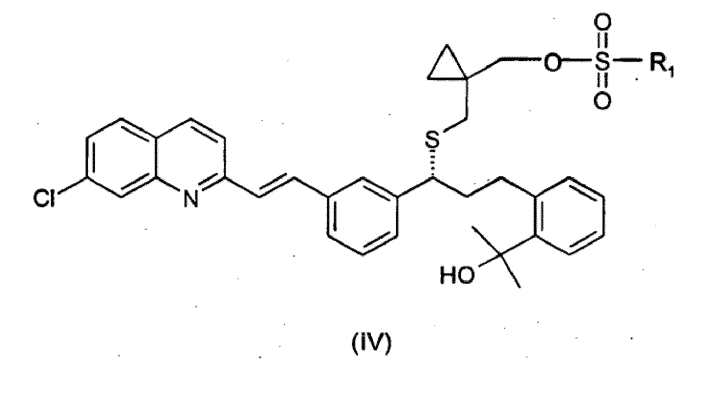 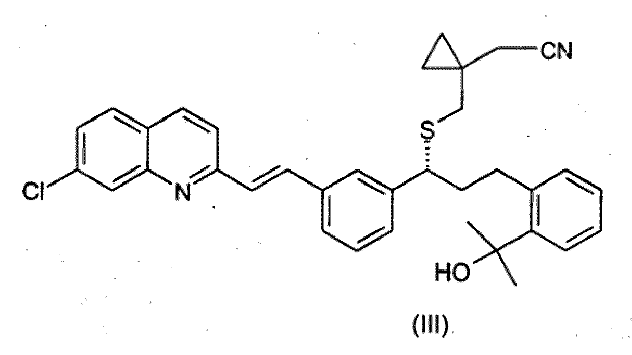
(b) Gegebenenfalls Hydrolysieren der aus Schritt (a) hervorgegangenen Verbindung der Formel (III) unter Reaktionsbedingungen, die zu einer Verbindung der Formel (II) führen, und Isolieren der besagten Verbindung (II) vom Reaktionsmedium; 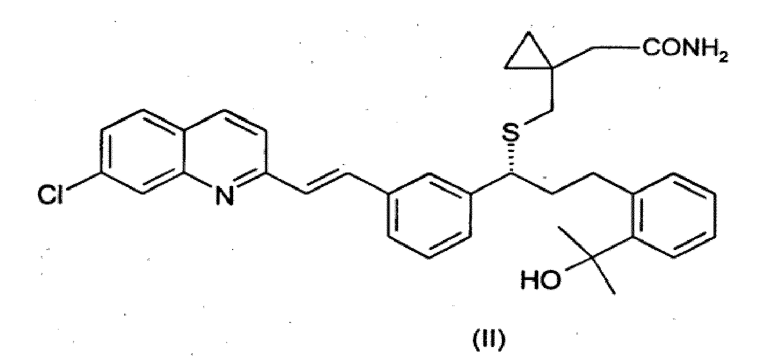
(c) Durchführen einer Hydrolysereaktion an der aus Schritt (a) oder aus Schritt (b) hervorgegangenen Verbindung unter Reaktionsbedingungen, die zu Montelukast (I) führen; und
(d) gegebenenfalls Behandlung des Montelukasts (I) mit einer pharmazeutisch verträglichen Base, um das korrespondierende Salz zu bilden.

2. Herstellungsverfahren nach Anspruch 1, bei dem die Verbindung der Formel (III) in Form einer freien Base isoliert wird.

3. Herstellungsverfahren nach einem der Ansprüche 1-2, wobei R₁ aus der Gruppe ausgewählt wird, die aus Methyl, Phenyl und 4-Methylphenyl besteht.

4. Herstellungsverfahren nach einem der Ansprüche 1-3, wobei Schritt (a) in Gegenwart eines Phasentransferkatalysators ausgeführt wird.

5. Herstellungsverfahren nach einem der Ansprüche 1-4, wobei die Hydrolysereaktion der Schritte (b) und (c) mit einer Base ausgeführt wird.

6. Herstellungsverfahren nach Anspruch 5, wobei die Hydrolyse in einer Mischung, die Wasser und ein organisches Lösungsmittel enthält, gegebenenfalls in Gegenwart eines Phasentransferkatalysators, durchgeführt wird.

7. Herstellungsverfahren nach einem der Ansprüche 1-6, bei dem in einem weiteren Schritt eine Verbindung der Formel (V) mit einem Sulfonsäurechlorid der Formel Cl-SO₂-R₁, worin R₁ dieselbe Bedeutung wie in Anspruch 1 hat, zur Reaktion gebracht wird, um eine Verbindung der Formel (IV) zu ergeben.

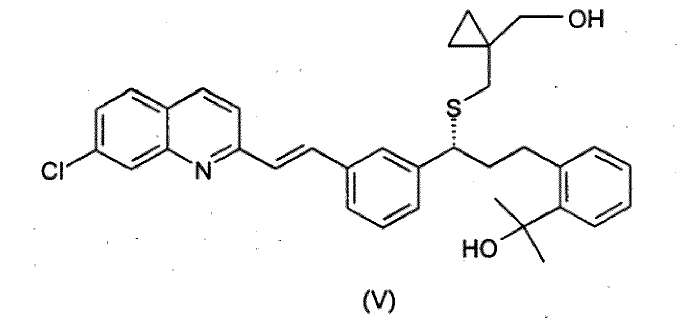

8. Herstellungsverfahren nach Anspruch 7, bei dem in einem weiteren Schritt eine Verbindung der Formel (VI) mit einem Grignardreagens, das aus einem Methyllithiumhalogenid und einem Methylmagnesiumhalogenid ausgewählt wird, zur Reaktion gebracht wird, gegebenenfalls in Gegenwart von Cerchlorid, um eine Verbindung der Formel (V) zu ergeben, und die besagte Verbindung (V) in Form einer freien Base oder eines Salzes davon isoliert wird.

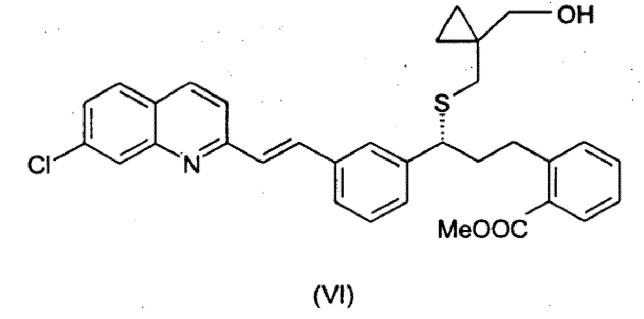

9. Herstellungsverfahren nach Anspruch 8, bei dem die Verbindung der Formel (V) in Form einer freien Base isoliert wird,

10. Herstellungsverfahren nach einem der Ansprüche 8-9, bei dem in einem weiteren Schritt die Verbindung der Formel (VII), worin R₂ ein Radikal ist, das aus der Gruppe, die aus (C₁-C₄)-Alkyl, Phenyl, und ein- oder zweifach mit (C₁-C₄)-Alkylradikalen substituiertem Phenyl besteht, ausgewählt wird, mit einer Verbindung der Formel (VIII) in Gegenwart einer Base zur Reaktion gebracht wird, um eine Verbindung der Formel (VI) zu ergeben, und die besagte Verbindung (VI) in Form einer freien Base oder eines Salzes davon isoliert wird.

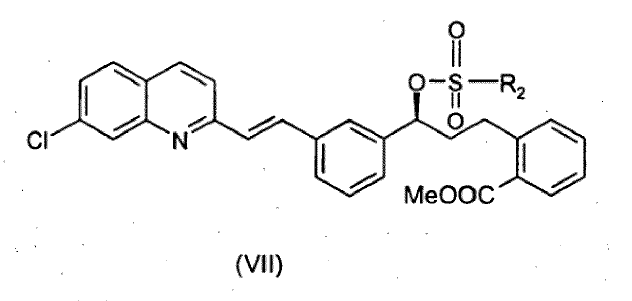

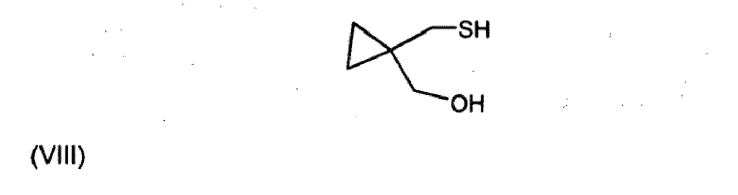

11. Herstellungsverfahren nach Anspruch 10, bei dem die Verbindung der Formel (VI) in Form einer freien Base isoliert wird.

12. Herstellungsverfahren nach Anspruch 10, bei dem die Verbindung der Formel (VI) in Form eines Oxalatsalzes isoliert wird.

13. Herstellungsverfahren nach einem der Ansprüche 10-12, wobei R₂ aus der Gruppe ausgewählt wird, die aus Methyl, Phenyl und 4-Methylphenyl besteht.

14. Verbindung der Formel (IV), worin R₁ ein Radikal ist, das aus der Gruppe, die aus (C₁-C₄)-Alkyl, Phenyl, und ein- oder zweifach mit einem (C₁-C₄)-Alkylradikal substituiertem Phenyl besteht, ausgewählt wird.

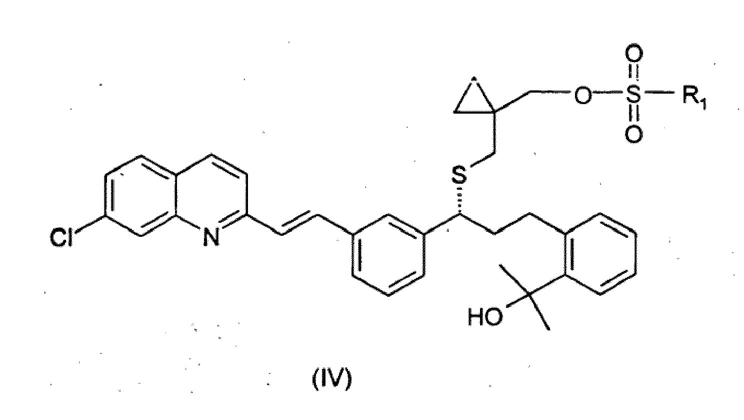

15. Verbindung nach Anspruch 14, wobei R₁ aus Methyl, Phenyl und 4-Methylphenyl ausgewählt wird.

16. Verbindung der Formel (V) oder ein Salz davon.

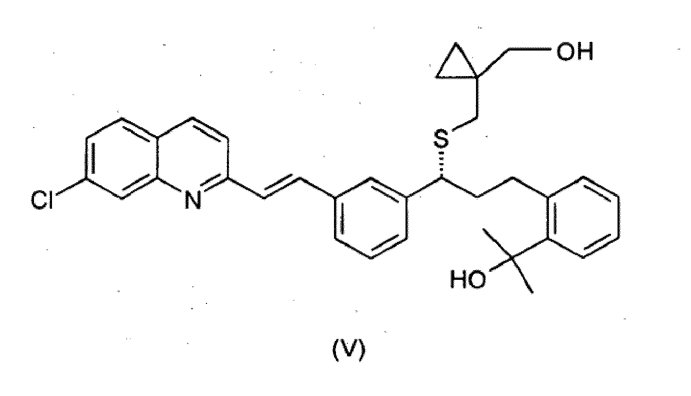

17. Verbindung nach Anspruch 16, worin das Salz das Citrat ist.

18. Verbindung der Formel (VI) oder ein Salz davon.

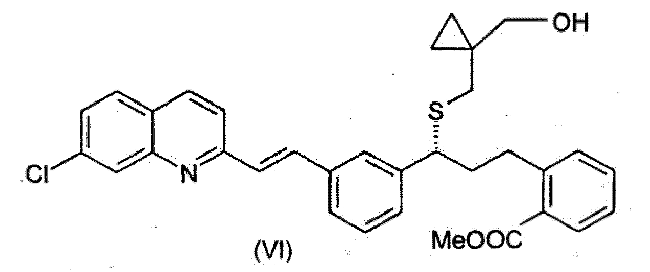

19. Verbindung nach Anspruch 18, worin das Salz das Oxalat ist.

20. Ein Salz der Verbindung der Formel (III), das aus der Gruppe ausgewählt wird, die aus Oxalat, L-(-)-Malat, L-(-)-Tartrat, Maleat, Fumarat, Succinat, Benzoat, 4-Hydroxymandelat, Citrat, Benzensulfonat und Mandelat besteht.

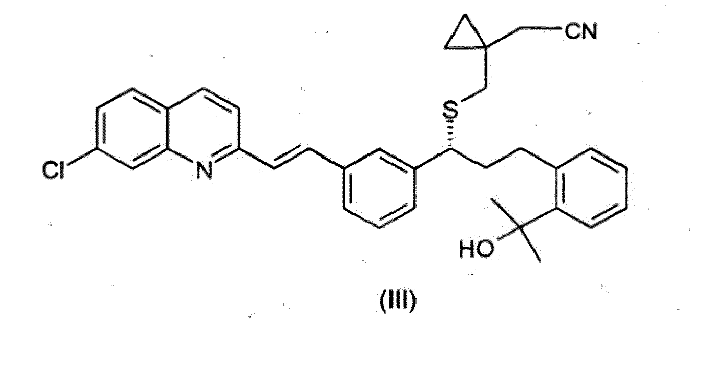

## Revendications

1. Procédé de préparation de Montélukast (I), ou un sel acceptable sur le plan pharmaceutique, ou un solvate de celui-ci, y compris un hydrate. 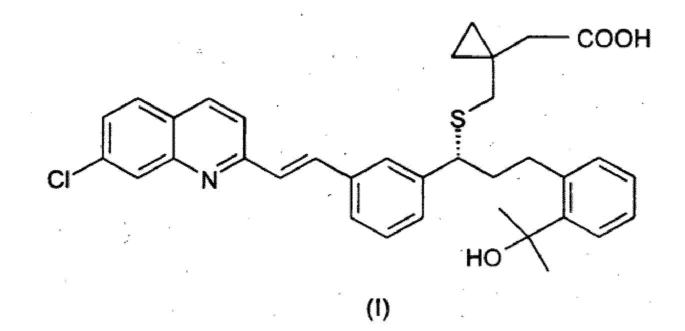 qui comprend les étapes consistant à :
(a) faire réagir un composé de formule (IV), dans laquelle R₁ est un radical choisi dans le groupe constitué d'un alkyle en (C₁ à C₄), un phényle, et un phényle mono ou disubstitué par un radical alkyle en (C₁ à C₄), avec un cyanure de métal alcalin, pour donner un composé de formule (III) et isoler ledit composé (III) en tant que base libre ou en tant que sel de celle-ci ; 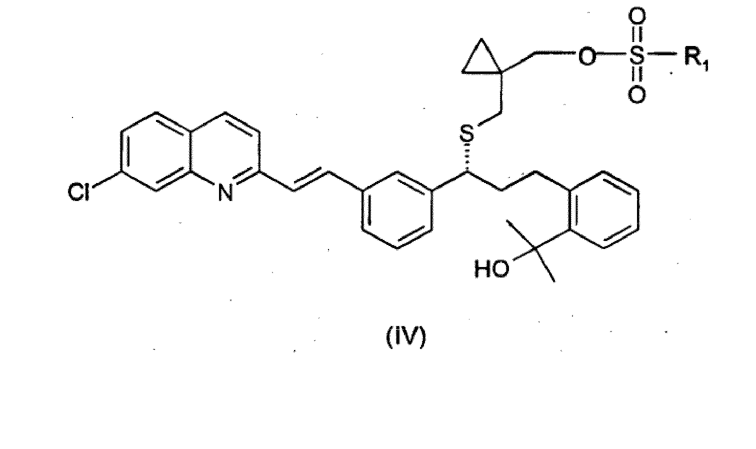 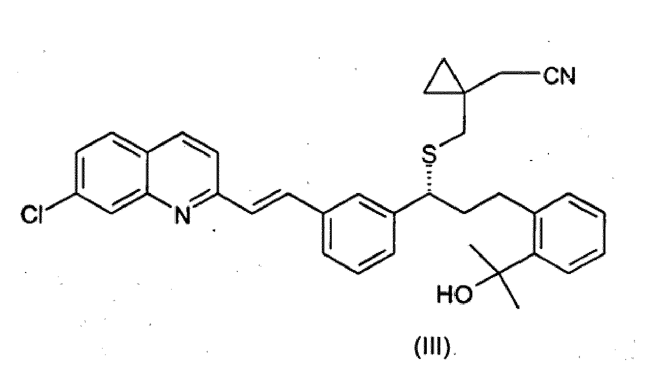
(b) éventuellement, hydrolyser le composé de formule (III) obtenu dans l'étape (a) dans des conditions de réaction qui donnent un composé de formule (II) et isoler ledit composé (II) du milieu réactionnel ; 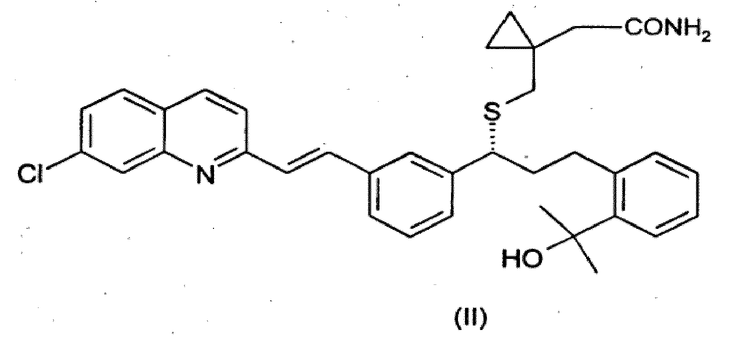
(c) soumettre le composé obtenu dans l'étape (a) ou dans l'étape (b) à une réaction d'hydrolyse dans des conditions de réaction qui donnent du Montélukast (I) ; et
(d) éventuellement traiter le Montélukast (I) avec une base acceptable sur le plan pharmaceutique pour former le sel correspondant.

2. Procédé de préparation selon la revendication 1, dans lequel le composé de formule (III) est isolé en tant que base libre.

3. Procédé de préparation selon l'une quelconque des revendications 1 à 2, dans lequel R₁ est choisi dans le groupe constitué de méthyle, phényle, et 4-méthylphényle.

4. Procédé de préparation selon l'une quelconque des revendications 1 à 3, dans lequel l'étape (a) est effectuée en présence d'un catalyseur de transfert de phase.

5. Procédé de préparation selon l'une quelconque des revendications 1 à 4, dans lequel la réaction d'hydrolyse des étapes (b) et (c) est effectuée avec une base.

6. Procédé de préparation selon la revendication 5, dans lequel l'hydrolyse est effectuée dans un mélange comprenant de l'eau et un solvant organique, éventuellement en présence d'un catalyseur de transfert de phase.

7. Procédé de préparation selon l'une quelconque des revendications 1 à 6, dans lequel dans une étape ultérieure, un composé de formule (V) est mis en réaction avec un chlorure de sulfonyle de formule Cl-SO₂-R₁, dans laquelle R₁ a la même signification que dans la revendication 1 pour donner un composé de formule (IV).

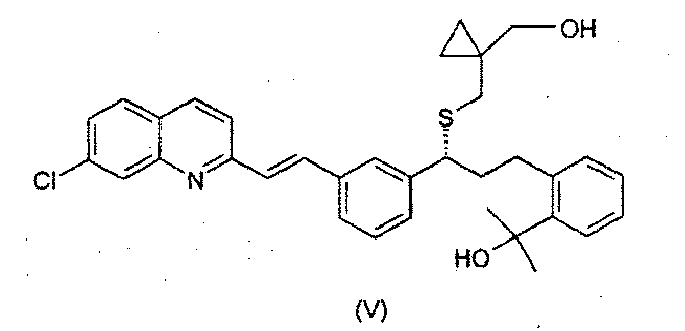

8. Procédé de préparation selon la revendication 7, dans lequel dans une étape ultérieure, un composé de formule (VI) est mis en réaction avec un réactif de Grignard choisi parmi un halogénure de méthyl-lithium et un halogénure de méthyl-magnésium, éventuellement en présence de chlorure de cérium pour donner un composé de formule (V) et ledit composé (V) est isolé en tant que base libre ou en tant que sel de celle-ci.

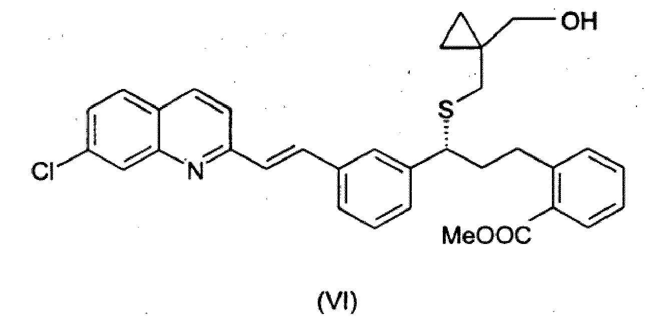

9. Procédé de préparation selon la revendication 8, dans lequel le composé de formule (V) est isolé en tant que base libre.

10. Procédé de préparation selon l'une quelconque des revendications 8 à 9, dans lequel dans une étape ultérieure le composé de formule (VII), dans laquelle R₂ est un radical choisi dans le groupe constitué d'un alkyle en (C₁ à C₄), un phényle, et un phényle mono ou disubstitué par des radicaux alkyle en (C₁ à C₄), est mis en réaction avec un composé de formule (VIII), en présence d'une base pour donner un composé de formule (VI) et ledit composé (VI) est isolé en tant que base libre ou en tant que sel de celle-ci.

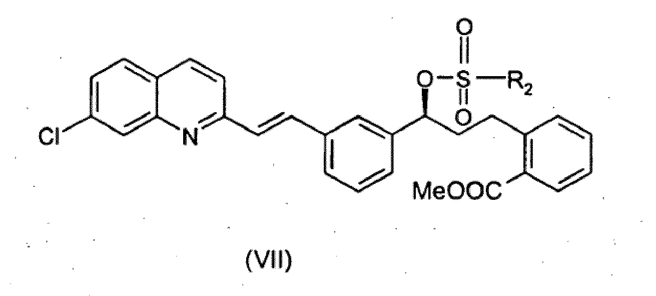

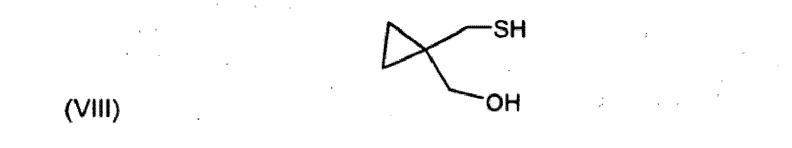

11. Procédé de préparation selon la revendication 10, dans lequel le composé de formule (VI) est isolé en tant que base libre.

12. Procédé de préparation selon la revendication 10, dans lequel le composé (VI) est isolé en tant que sel d'oxalate.

13. Procédé de préparation selon l'une quelconque des revendications 10 à 12, dans lequel R₂ est choisi dans le groupe constitué de méthyle, phényle, et 4-méthylphényle.

14. Composé de formule (IV), dans laquelle R₁ est un radical choisi dans le groupe constitué d'un alkyle en (C₁ à C₄), un phényle, et un phényle mono ou disubstitué par un radical alkyle en (C₁ à C₄).

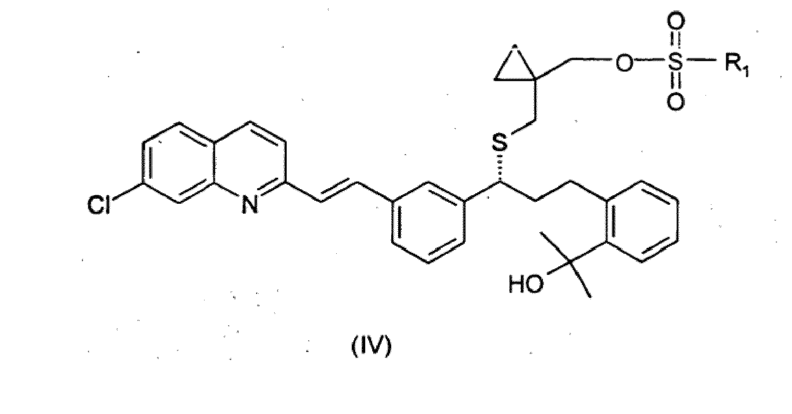

15. Composé selon la revendication 14, dans lequel R₁ est choisi parmi méthyle, phényle, et 4-méthylphényle.

16. Composé de formule (V) ou un sel de celui-ci.

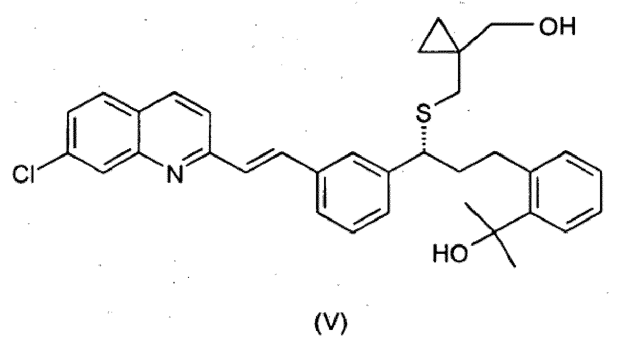

17. Composé selon la revendication 16, dans lequel le sel est le citrate.

18. Composé de formule (VI) ou un sel de celui-ci.

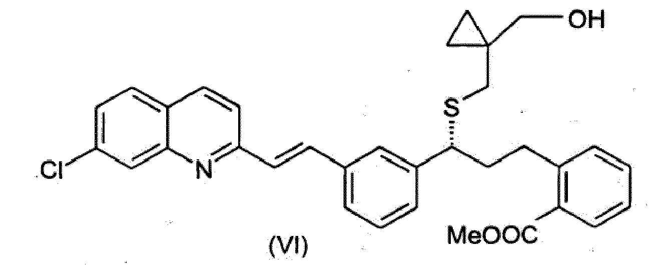

19. Composé selon la revendication 18, dans lequel le sel est l'oxalate.

20. Un sel du composé de formule (III) qui est choisi dans le groupe constitué d'oxalate, L-(-)-malate, L-(-)-tartrate, maléate, fumarate, succinate, benzoate, 4-hydroxymandélate, citrate, benzènesulfonate, et mandélate.
